# EUROPEAN PATENT SPECIFICATION

(11) **EP 2 247 621 B1**
(45) Date of publication and mention of the grant of the patent: **27.04.2016**
(21) Application number: 09714192.3
(22) Date of filing: 23.02.2009
(51) Int. Cl.: C07K 16/40, C07K 14/47

(54) **MONOSPECIFIC ANTIBODY AND METHOD OF PRODUCTION USING AS ANTIGEN AN ISOFORM OF THE HUMAN FAD SYNTHETASE.**
MONOSPEZIFISCHER ANTIKÖRPER UND HERSTELLUNGSVERFAHREN, BEI DEM ALS ANTIGEN EINE ISOFORM DER HUMANEN FAD-SYNTHETASE VERWENDET WIRD
ANTICORPS MONOSPÉCIFIQUE ET PROCÉDÉ DE PRODUCTION UTILISANT COMME ANTIGÈNE UNE ISOFORME DE LA FAD SYNTHÉTASE HUMAINE

(30) Priority: 26.02.2008 IT CZ20080004
(43) Date of publication of application: 10.11.2010
(73) Proprietor: Universita Della Calabria, 87036 Rende (CS) (IT); Università degli Studi di Bari "Aldo Moro", 70121 Bari (BA) (IT)
(72) Inventor: BARILE, Maria, I-70126 Bari (IT); TORCHETTI, Enza Maria, I-70126 Bari (IT); INDIVERI, Cesare, I-87036 Rende (IT); GALLUCCIO, Michele, I-87036 Rende (IT)
(74) Representative: Di Giovine, Paolo
(86) International application number: PCT/IT2009/000062
(87) International publication number: WO 2009/107158

(56) References cited:
- GALLUCCIO ET AL: "Over-expression in Escherichia coli, purification and characterization of isoform 2 of human FAD synthetase" PROTEIN EXPRESSION AND PURIFICATION, ACADEMIC PRESS, SAN DIEGO, CA, vol. 52, no. 1, 8 January 2007 (2007-01-08), pages 175-181, XP005758621 ISSN: 1046-5928 cited in the application
- BRIZIO C ET AL: "Over-expression in Escherichia coli and characterization of two recombinant isoforms of human FAD synthetase" BIOCHEMICAL AND BIOPHYSICAL RESEARCH COMMUNICATIONS, ACADEMIC PRESS INC. ORLANDO, FL, US, vol. 344, no. 3, 9 June 2006 (2006-06-09), pages 1008-1016, XP024924993 ISSN: 0006-291X [retrieved on 2006-06-09] cited in the application
- TORCHETTI E M ET AL: "Mitochondrial localization of human FAD synthetase isoform 1", MITOCHONDRION, ELSEVIER, AMSTERDAM, NL, vol. 10, no. 3, 1 April 2010 (2010-04-01), pages 263-273, XP026980512, ISSN: 1567-7249 [retrieved on 2010-01-07]

## Description

### Technical field of invention

The FAD (Flavin Adenin Dinucleotide) protein synthetase is an enzyme which catalyses an obligatory and ubiquitous phase of the energetic metabolism, namely the ATP adenylazation depending on flavin mononucleotide or FMN, which in its turn derives from the B₂ vitamin or riboflavin. The procaryotic and eucaryotic cells use the FAD as co-maker of hundreds of different flavoenzymes with dehydrogenasic and oxydasic activities. These are involved in the terminal metabolism, in the processes of energetic transduction, in protection from the cellular stress, in folding of the secretion protein, in the independent caspase apoptosis, in the chromatin remodeling processes, etc.

The FAD synthetase has a remarkable importance as potential target of the pharmacological therapy because the human protein indeed is structurally different from those bacterical and from the FAD synthetase of the *Plasmodium ƒalciparum,* pathological agent of the malaria.

The antibodies obtained from the serum of laboratory animals in which the FAD synthetase antigen was injected, can be employed mainly in the research field in order to clarify the aspects of the FAD synthetase cellular biochemistry not yet solved (expression variations, other isoforms existence, tissues specificity, organs specificity etc). The developments which can be previewed in the field of clinics and diagnostics deserve to be mentioned too.

Some fisio-pathological aspects correlated to the FAD enzyme synthetase are still object of research. For example, one of its alteration can be put in relation to multienzymetic mitochondrial disfunctions like RR-MADD (riboflavin responsive multiple acilCoA dehydrogenase deficiencies), a heavy neuromuscular pathology which regresses under treating with pharmacological doses of riboflavin. The FAD synthetase identification in human muscle and its quantification in the multienzymatic deficits of the flavoenzymes will be possible with the usage of the new antiserum.

The riboflavin absorbing depends on the activity of the FAD entherosyte synthetase and the ratio of the current flavins depends on erythtrocyte one. The nutrient state of FAD influences of the folate level, of other hydrosoluble vitamins and of current homocystein with pathological consequences for the cardio-circulatory system. In particular, the troubles in the FAD synthesis are associated to hyperthyroidism, the state in which the cardiovascular risks grow. The usage of the antibody will be useful to measure the nutritional state of flavine co-factors in different tissues and, in particular, it could have high diagnostical value for the identification of the FAD synthetase in the thyroid tissues and its quantification in the hypothyroidism.

The interest of the antibodies, directed towards the human FAD synthetase enzyme, in pharmacological field is given by the possibility to use an antibody for the identification and the quantitative measure of the enzyme in cellular extracts, biological liquids and biopsies in different fields of application: 1) there exist human pathologies which could be caused by the deficiency of the FAD synthetase protein, like the RR-MADD, which if it is diagnosticated in time can be treated with the simple ingestion of riboflavine in high doses. The antibody could make possible the precocious diagnosis. 2') There is a deep difference between the correspondent protein of the bacterium (bacterial FAD synthetase) and the human one. Being both of them important for the vital functions of the organisms, both bacterial and human, the bacterial protein constitutes a potential site of attack for planning of new generation antibiotics which would have the action to inactivate only the bacterial protein oppressing the bacteria during the infective process, without interfering with the vital functions of human organism. The pharmaceutical industries are very interested to this aspect. The antiserum would permit the monitoring of the human protein. 3) Planning and production of diagnostic kits or microchips for recognizing of the FAD synthetase protein on the support for the applications in molecular diagnostics and in pharmacological research.

### State of the art

Up to nowadays the measuring of the FAD synthetase activity was conducted with spectrofluorimetric techniques in enzymatic *catalytic in continuo* dosages which realization requests time for samples preparation and specific competences of enzymatic kinetics. As an alternative, high resolution liquid chromatography, a technique which requests, first of all, the usage of sophisticated and dear apparatuses, is used.

The production of an antibody obtained from the FAD synthetase antigen permits a rapid and simple research of the alterations of the same FAD synthetase usable in immuno-electrophoretic or immuno-enzymatic applications.

In particular, as far as the over-expression of the FAD protein is concerned, the following scientific publications are noted:
- Brizio C, Galluccio M, Wait R, Torchetti EM, Bafunno V, Accardi R, Gianazza E, Indiveri C, Barile M. Over-expression in Escherichia coli and characterization of two recombinant isoforms of human FAD synthetase. Biochem Biophys Res Commun. 2006; 344(3):1008-16;
- Galluccio M, Brizio C, Torchetti EM, Ferranti P, Gianazza E, Indiveri C, Barile M. Over-expression in Escherichia coli, purification and characterization of isoform 2 of human FAD synthetase. Protein Expr Purif. 2007 Mar;52175-81;
which describe the method according to which two isoforms of the human FAD synthetase have been cloned and over-expressed in the bacteria.

The patent EP0542240 describes a process for the synthesis of FMN and/or FAD by means of technology of recombinant DNA in microorganisms cultures, *E. coli* included.

The patent EP0146871 describes a method of purification of the FAD synthetase protein by means of chromatographic column and the JP6112772661 refers to a method of purification of an antigene by means of electrophoresis which supposes the usage of a polyacrylamide gel sublayer to enhance the antibody feedback of the animal but in both the purification and solubilization of the human antigen FAD synthetase necessary in the phase of inoculation in the test animal are not previewed.

The main aim of the present invention is that to overcome the difficulties and the disadvantages present in the state of the art realizing a method of production of antibodies obtained using as antigen an isoform of the human FAD synthetase , obtained by means of technology of recombinant DNA cloned and over-expressed in microorganisms cultures, characterized by the fact that said antigen is purified and concentrated by means of electrophoresis and extraction in tampon before being inoculated in a test animal.

Another characteristics is given by the fact that the used culture of microorganisms is constituted by a colony of *E. coli Rosetta (DE3)* cells.

Another characteristics is given by the fact that the isoform 2 of the human FAD synthetase is used as antigen.

Another characteristics is given by the fact that the synthesis of the isoform 2 of the human FAD synthetase by means of the IPTG (isopropyl-β-D-thiogalactopyranoside) adding to the concentration of 0.4 mM, is inducted when the optical density of the culture measured on the 600 nm wave length has achieved the 0.57 value.

Another characteristics is given by the fact that after the induction period which varies from 4 to 6 hours the bacterial culture is sedimented by means of centrifugation at values from 2800 to 3200 rpm at the temperature which varies from 2 to 8 °C.

Another characteristics is given by the fact that the bacterial sediment equal to more or less 3 g is reweightened in an opportune buffer at pH 7.3 containing 4.29 mM Na₂HPO₄ 1.47mM KH₂PO₄, 2.7mM KCl, 137mM NaCl, 0.1% Tween 20, 0.002% sodium azide and sonicate for 5 minutes at 0°C by means of 40 Watt to lyse the cells and extract the proteins.

Another characteristics is given by the fact that the *E. coli* cells lysate in which the synthesis of the protein human FAD synthetase isoform 2 with 400 µl of sample buffer is charged on gel of polyacrylamide in sodium dodecylsulphate (SDS) at 12 %.

Another characteristics is given by the fact that the proteins are separated in base of their dimensions thanks to the electric charge of the protein conferred by the sodium dodecylsulphate (SDS) and to the mass of the protein in the passage through the gel.

Another characteristics is given by the fact that it is previewed the extraction of the FAD synthetase protein by means of immersion in 1.5 ml of a buffer containing NaCl 70mM, KH₂PO₄ 0.75 mM, Na₂HPO₄ 5.08 mM, KCl 1.35 mM, SDS (sodium dodecylsulphate) 0.3% added of Tris to bring the pH of the suspension to pH 8.0.

Another main aim of the invention is that to realize an antibody obtained by means of usage of an isoform of the human FAD synthetase as antigen.

### Detailed description of an embodiment of the invention

### 1. Production of the human recombinant FAD synthetase isoform 2

A colony of cells *E. coli Rosetta (DE3)* transformed with the recombinant FAD synthetase isoform 2-pET-21a(+) construct is put into 400 ml of LB culture broth at the temperature of 30°C containing ampicillin at 100 µg/ml concentration and chloramphenicol at 34 µg/ml concentration. When the optical density of the culture measured at 600 nm has achieved the value of 0.57, the synthesis of the isoform 2 of the human FAD synthetase is induced by means of the adding of IPTG (isopropyl-β-D-thiogalactopyranoside) at 0.4 mM concentration. After 4 hours of bacterial induction the bacterial culture is sedimented by means of centrifugation at 3000 rpm at the temperature of 4°C. The bacterial sediment equal to approximately 3 g is reweightened in an opportune buffer at pH 7.3 containing 4.29 mM Na₂HPO₄, 1.47mM KH₂PO₄, 2.7mM KCl, 137mM NaCl, 0.1% Tween 20, 0.002% sodium azide and sonicate for 5 minutes at 0°C with 40 Watt to lyse the cells and extract the proteins..

### 2. Concentration and purification of the FAD synthetase antigen by means of electrophoresis and extraction in buffer

On two gels of polyacrylamide in sodium dodecylsulphate (SDS) at 12 %, 20 identical samples obtained by reweightening the sediment of 200 µl of the of E. coli lysate cells in which the synthesis of the human protein FAD synthetase isoform 2 with 400 µl of sample buffer was induced are charged. The proteins are separated in base of their dimensions and after an electrophoretic course the gel is coloured with Blue Coomassie.

The bands corresponding to the protein FAD synthetase are cut and the obtained pieces of gel are immerged in 1.5 ml of a buffer containing NaCl 70mM, KH₂PO₄ 0.75 mM, Na₂HPO₄ 5.08 mM, KCl 1.35 mM, SDS (sodium dodecylsulphate) 0.3% added of Tris to bring the pH of the suspension to pH 8.0. The suspension is kept under shaking. After 12 h the solution is removed leaving the pieces of gel in the test tube to which 1.5 ml of fresh solution identical to the previous one are added. After other 12 h the second solution is removed and joined to the first one. The purified and concentrated protein is precipitated in acetone and solubilized in 0.4 ml of the extraction tampon used before. The protein extracted in tampon containing 0.3% of sodium dodecylsulphate, which enhances the animal antibody feedback is treated with the complete Freund's adiuvant containing the inactivated *Mycobacterium tubercolosis* in proportion 1:1 and injected in a New Zealand albino male rabbit.

The Freund's adiuvant is a mix of mineral oil, an emulsionant agent and a suspension of inactivated *Mycobacterium tubercolosis.* The mineral oil provokes a slow release of the antigen from the emulsion and the mycobacterium activates the immune system provoking the formation of a granuloma. After the two first injections a third intravenous administration is executed and subsequently, after one week, the blood intaking by means of ear marginal vein incision is executed. The obtained blood intaking provides an immune serum containing antibodies directed against the antigen. In particular, for the production of the antibody it was chosen to use the entire antigen (FAD synthetase 2) and not small pieces (peptides) of the same, in order to obtain a more efficient antiserum in comparison to that obtained by means of the procedure which previews the usage of the protein pieces. The major efficiency and specificity is determined, probably, by the presence of more than one specific epitopes on the entire protein and by its major immunogenicity.

The antiserum produced in such a way was tested for the identification of the recombinant enzyme FAD synthetase by means of the immuno-blotting technique. It consists in the transfer of proteins from one gelatinous matrix (polyacrylamide gel in sodium dodecylsulphate) to a membrane of nitrocellulose on which surface the proteins adher. Subsequently the protein recognized by the antibody can be evidenced on the surface of the membrane by means of enzymatic coloring.

The analysis executed by means of western blotting indicates clearly that the produced antibody is highly specific to the human enzyme FAD synthetase (both to the isoform 1 and 2).

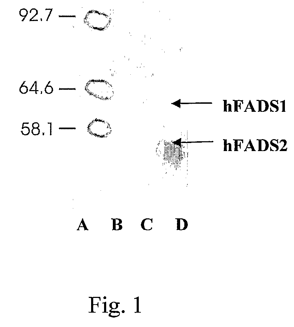

In fact, the figure 1 shows a typical electrophoretogram obtained with antiserum diluted 1:5000 and 0.5 and 2 micrograms of the two recombinant proteins (lane C e D respectively). The lanes are identified by means of the colorimetric relevation using a secondary commercial antibody conjugated with the alkaline phosphatase.

The specificity of the antiserum anti-FAD synthetase is proved by the lack of the immuno-reaction with the albumin protein of bovine serum (lane B) or other marker proteins charged in equimolecular quantity for control (lane A).

In base to the high specificity and immuno-reactivity of the produced antiserum, this is considered a powerful mean for the diagnostical screening of pathologies and for the identification of the enzyme also when it is present in very low quantities in different preparations.

The antiserum produced in this invention, for example, reacts in a specific manner against the human FAD synthetase protein contained in human hemolysates obtained with protocols generally in use in chemical analysis laboratories.

The monospecific antibody against the obtained enzyme human FAD synthetase represents the only molecular test available for the identification of the human and the rat's FAD synthetase protein.

## Claims

1. A method for producing antibodies obtained using as antigen the isoform 2 of the human FAD synthetase, said method comprising the following steps:
- cloning and over-expressing said isoform 2 of the human FAD synthetase in microorganism cultures;
- culturing said cells up to an optic density of 0.57 OD at 600 nm;
- inducing the synthesis of said isoform 2 for a period of 4-6 hours by adding 0.4 mM IPTG (isopropyl-ß-D-thiogalactopyranoside);
- sedimenting the induced cells and suspending said cells, which are equal more or less to 3 gr, in a buffer with a pH= 7.3 and containing 4.29 mM Na₂HPO₄, 1.47 mM KH₂PO₄, 2.7 mM KCl, 137mM NaCl, 0.1 % Tween 20, 0.002% azide
- carrying out a sonication of the suspended cells at 0°C at 40 Watt for 5 minutes;
- purifying and concentrating said isoform 2 of the human FAD synthetase using gel electrophoresis technique;
- extracting said isoform 2 of the human FAD synthetase using 1.5 ml buffer containing NaCl 70 mM, KH₂PO₄ 0.75 mM, Na₂HPO₄ 5.08 mM, KCL 1.35 mM, sodium dodecylsulphate 0.3% added of Tris to bring the pH of the suspension to PH 8.0;
- precipitating said isoform 2 in acetone and then solubilizing it in 0.4 ml of said extraction buffer comprising sodium dodecylsulphate 0.3%;
- mixing said extracting buffer comprising the extracted protein with a complete Freund's adjuvant containing a inactivated *Mycobacterium tuberculosis* in a proportion of 1:1;
- inoculating the mix to a non-human animal;
- obtaining blood from said non-human animal, which blood provides an immune serum containing antibodies directed to said antigen.

2. The method according to claim 1, **characterized by** the fact that said culture of microorganisms is constituted by a colony of *E.coli Rosetta (DE3) cells.*

3. The method according to any one of claims 1-2, wherein the step of sedimentation is carried out by means of centrifugation at values from 2800 to 3200 rpm at the temperature which varied from 2 to 8 °C.

4. The method according to any one of claims 1-3, wherein said gel electrophoresis is carried out on a gel of polyacrylamide in sodium dodecylsulphate (SDS) at 12%

5. The method according to any one of claims 1-4, wherein said animal is a New Zeeland albino male rabbit.

6. Immune serum containing antibodies obtainable by the method according to any one of claims 1-5 **characterized in that** said antibodies are polyclonal and are capable of recognizing both isoform 1 and isoform 2 of the human FAD synthetase protein.

## Patentansprüche

1. Verfahren zur Herstellung von Antikörpern, die unter Verwendung der Isoform 2 der humanen FAD-Synthetase als Antigen erhalten werden, wobei das Verfahren die folgenden Schritte umfasst:
- Klonieren und Überexprimieren der Isoform 2 der humanen FAD-Synthetase in Mikroorganismenkulturen;
- Züchten der Zellen bis zu einer optischen Dichte von 0,57 OD bei 600 nm;
- Induzieren der Synthese der Isoform 2 für einen Zeitraum von 4 bis 6 Stunden durch Zugeben von 0,4 mM IPTG (Isopropyl-β-D-thiogalactopyranosid);
- Sedimentieren der induzierten Zellen und Suspendieren der Zellen, die mehr oder weniger gleich 3 g sind, in einem Puffer mit einem pH-Wert von 7,3 und der 4,29 mM Na₂HPO₄ 1,47 mM KH₂PO₄ 2,7 mM KCl, 137 mM NaCl, 0,1% Tween 20, 0,002% Azid enthält;
- Durchführen einer Ultraschallbehandlung der suspendierten Zellen bei 0°C bei 40 Watt für 5 Minuten;
- Aufreinigen und Konzentrieren der Isoform 2 der humanen FAD-Synthetase unter Verwendung von Gelelektrophorese-Verfahren;
- Extrahieren der Isoform 2 der humanen FAD-Synthetase unter Verwendung von 1,5 ml Puffer enthaltend NaCl 70 mM, KH₂PO₄ 0,75 mM, Na₂HPO₄ 5,08 mM, KCl 1,35mM, Natriumdodecylsulfat 0,3%, wobei Tris zugegeben ist, um den pH-Wert der Suspension auf pH 8,0 zu bringen;
- Präzipitieren der Isoform 2 in Aceton und anschließende Solubilisierung dieser in 0,4 ml Extraktionspuffer umfassend Natriumdodecylsulfat 0,3%:
- Vermischen des Extraktionspuffers, der das extrahierte Protein umfasst, in einem 1:1 Verhältnis mit einem kompletten Freund-Adjuvans, das ein inaktiviertes *Mycobacterium tuberculosis* enthält;
- Impfen eines nicht-menschlichen Tieres mit dem Gemisch;
- Entnehmen von Blut von dem nicht-menschlichen Tier, dessen Blut ein Immunserum bereitstellt, das Antikörper enthält, die gegen das Antigen gerichtet sind.

2. Verfahren nach Anspruch 1, **gekennzeichnet durch** die Tatsache, dass die Kultur von Mikroorganismen **durch** eine Kolonie von *E.coli Rosetta(DE3)*-Zellen begründet ist.

3. Verfahren nach einem der Ansprüche 1 bis 2, wobei der Schritt des Sedimentierens mittels Zentrifugation bei Werten von 2800 bis 3200 rpm bei einer Temperatur, die zwischen 2 bis 8°C variiert, durchgeführt wird.

4. Verfahren nach einem der Ansprüche 1 bis 3, wobei die Gelelektrophorese in einem Polyacrylamid-Gel in Natriumdodecylsulfat (SDS) bei 12% durchgeführt wird.

5. Verfahren nach einem der Ansprüche 1 bis 4, wobei das Tier ein männliches Neuseeland-Albinokaninchen ist.

6. Immunserum, das Antikörper erhältlich durch das Verfahren nach einem der Ansprüche 1 bis 5 enthält, **dadurch gekennzeichnet, dass** die Antikörper polyclonal sind und in der Lage sind, beide, Isoform 1 und Isoform 2, des humanen FAD-Synthetase-Proteins zu erkennen.

## Revendications

1. Procédé de production d'anticorps obtenus au moyen de l'isoforme 2 de la FAD-synthétase humaine utilisée comme antigène, lequel procédé comporte les étapes suivantes :
- cloner et sur-exprimer ladite isoforme 2 de FAD-synthétase humaine dans des cultures de microorganismes ;
- cultiver lesdites cellules jusqu'à une densité optique (DO) de 0,57 à 600 nm ;
- induire la synthèse de ladite isoforme 2 pendant 4 à 6 heures, en ajoutant 0,4 mM d'IPTG (isopropyl-β-D-thiogalactopyranoside) ;
- faire sédimenter les cellules, après cette induction, et remettre ces cellules, qui font plus ou moins 3 g, en suspension dans un tampon à pH 7,3 qui contient 4,29 mM de Na₂HPO₄, 1,47 mM de KH₂PO₄, 2,7 mM de KCl, 137 mM de NaCl, 0,1 % de Tween 20 et 0,002 % d'azoture ;
- soumettre ces cellules en suspension à 5 minutes de sonication à 40 W et 0 °C ;
- purifier et concentrer ladite isoforme 2 de FAD-synthétase humaine par électrophorèse sur gel ;
- extraire ladite isoforme 2 de FAD-synthétase humaine au moyen de 1,5 mL de tampon contenant 70 mM de NaCl, 0,75 mM de KH₂PO₄, 5,08 mM de Na₂HPO₄, 1,35 mM de KCl, 0,3 % de docécyl-sulfate de sodium et additionné de Tris de manière à ce que le pH de la suspension soit ajusté à 8,0 ;
- faire précipiter ladite isoforme 2 dans de l'acétone, et la dissoudre ensuite dans 0,4 mL dudit tampon d'extraction contenant 0,3 % de dodécyl-sulfate de sodium ;
- mélanger ce tampon d'extraction, qui contient la protéine extraite, avec un adjuvant complet de Freund contenant des mycobactéries *Mycobacterium tuberculosis* inactivées, en un rapport de 1/1 ;
- inoculer le mélange à un animal non-humain ;
- et prélever du sang de cet animal non-humain, lequel sang fournit un immun-sérum contenant des anticorps dirigés contre ledit antigène.

2. Procédé conforme à la revendication 1, **caractérisé en ce que** ladite culture de microorganismes est constituée par une colonie de cellules *d'E. coli Rosetta (DE3).*

3. Procédé conforme à l'une des revendications 1 et 2, dans lequel l'étape de sédimentation est effectuée par centrifugation à une vitesse de 2800 à 3200 t/min, à une température qui varie de 2 à 8 °C.

4. Procédé conforme à l'une des revendications 1 à 3, dans lequel ladite électrophorèse sur gel est effectuée sur un gel de polyacrylamide à 12 % de docécyl-sulfate de sodium (SDS).

5. Procédé conforme à l'une des revendications 1 à 4, dans lequel ledit animal est un lapin albinos mâle New Zeeland.

6. Immun-sérum contenant des anticorps accessibles par un procédé conforme à l'une des revendications 1 à 5, **caractérisé en ce que** lesdits anticorps sont polyclonaux et sont capables de reconnaître tant l'isoforme 1 que l'isoforme 2 de la protéine FAD-synthétase humaine.
